# EUROPÄISCHE PATENTANMELDUNG

(11) **EP 2 942 076 A1**
(43) Veröffentlichungstag der Anmeldung: **11.11.2015**
(21) Anmeldenummer: 14003281.4
(22) Anmeldetag: 29.09.2014
(51) Int. Cl.: A61M 21/00, E04B 1/32, E04H 1/12, A61N 5/06, A61G 10/00

(54) **TORUSFÖRMIGER ENTSPANNUNGSRAUM**

(30) Priorität: 01.10.2013 CH 16852013
(71) Anmelder: Sonami AG, 9490 Vaduz (LI)
(72) Erfinder: Lathan, Daniel, 9490 Vaduz (LI)

(57) **Zusammenfassung**

Ein Entspannungsraum (1) mit zur Erzeugung verschiedenartiger Reize eines Menschen (2) ausgebildeten Einrichtungen (6) sowie mit einem begehbaren Innenraum (4) mit einer Innenrandfläche (5), wobei der Innenraum (4) die Topologie eines Ringtorus mit einem durchgehenden Torusloch (9) in der Mitte aufweist.

## Beschreibung

Die Erfindung betrifft einen von mehreren Menschen begehbaren Entspannungsraum, der im Innenraum mittels geeigneter Einrichtungen unübliche Empfindungen hervorruft, welche die Sinne des Menschen auf eine Art und Weise ansprechen, die Entspannungs- und Entstressungsvorgänge unterstützt. Nach der DE19814499C2 ist eine Wohlfühl-Sauna für den Privatbereich bekannt, die optische, olfaktorische, akustische und taktile Reize des Menschen anspricht. Die Aufgabe der Erfindung besteht in einer auch zur Verwendung im professionellen Wohlfühl-Bereich geeigneten Ausführung eines begehbaren Entspannungsraums. Die Aufgabe wird mit den Merkmalen des Anspruchs 1 gelöst. Vorteilhafte Weiterbildungen ergeben sich aus den Unteransprüchen.

Im Wesentlichen weist ein Entspannungsraum mit einem von mehreren Menschen begehbaren Innenraum, welcher Einrichtungen zur Erzeugung verschiedenartiger Reize des Menschen aufweist, die Topologie eines Ringtorus mit einem durchgehenden Torusloch in der Mitte auf.

Das nachfolgende, anhand der geschnittenen Figur erläuterte, Ausführungsbeispiel kombiniert sämtliche weiteren, optionalen Merkmale. So weist ein Entspannungsraum 1 einen von mehreren Menschen 2 durch eine nicht dargestellte Tür begehbaren Innenraum 4 auf, welcher die reifenförmige Topologie eines Ringtorus mit einem durchgehenden Torusloch 9 in der Mitte ausbildet. An dessen Innenrandfläche 5 sind Einrichtungen 6 in Form von Lichtelementen 6a, Lautsprechern 6b und Körperschallwandlern (Excitern) 6c zur Erzeugung optischer, akustischer und taktiler Reize der im Innenraum 4 befindlichen Menschen 2 vorhanden, die sich auf fest mit der Innenrandfläche 5 verbundenen Liegemöbeln 7 in Form von Liegen und Wannen entspannen. Die Innenrandfläche 5 des Ringtorus ist stückweise aus transparentem und transluzentem Material in Form von Plexiglas, Glas und ETFE-Folie zusammengesetzt, wobei das Torusloch 9 einteilig ausgeführt ist. Das im Umlaufsystem 15 mittels Pumpe 15a und Zuleitung 15b oben tangential ins Torusloch 9 einströmende Wasser 8 bildet darin einen, von einem unten angeordneten Propeller 10 zusätzlich verstärkten, Wirbel 11 aus und läuft zudem aussenseitig umfänglich über den frei auf einem Wasserfilm schwimmenden Entspannungsraum 1 ab. Der von oben und unten beleuchtete Wirbel 11 wird mit seinen Strömungsschlieren 12 an die Innenrandfläche 5 projiziert sowie, von einem Unterwassermikrofon 11 aufgenommen und geeignet gefiltert, von den Lichtelementen 6a, den Lautsprechern 6b und Körperschallwandlern (Excitern) 6c animiert wiedergegeben. Dieser Entspannungsraum 1 findet im professionellen Wohlfühl-Bereich kombiniert mit Entspannungs- und Stressmanagement-Methoden wie Massagen, Gymnastik, autogenes Training, Meditation, Kuren, Therapiegesprächen etc. Anwendung.

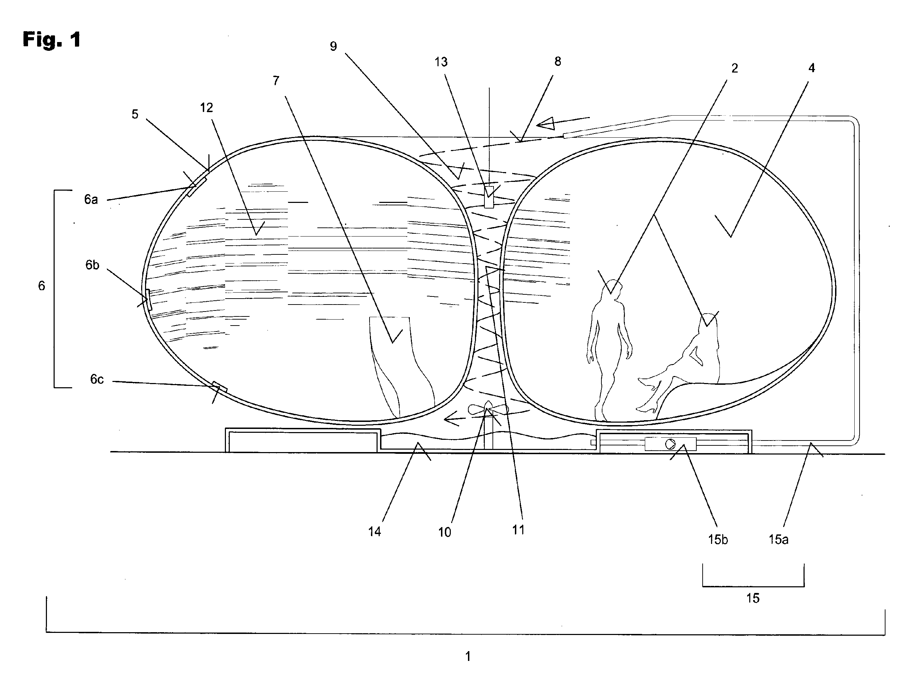

## Patentansprüche

1. Entspannungsraum mit zur Erzeugung verschiedenartiger Reize eines Menschen (2) ausgebildeten Einrichtungen sowie mit einem begehbaren Innenraum (4) mit einer Innenrandfläche (5), **dadurch gekennzeichnet, dass** der Innenraum (4) die Topologie eines Ringtorus mit einem durchgehenden Torusloch (9) in der Mitte aufweist.

2. Entspannungsraum nach Anspruch 1, **dadurch gekennzeichnet, dass** an der Innenrandfläche (5) geeignete Einrichtungen (6) zur Erzeugung optischer, akustischer und/oder taktiler Reize der im Innenraum (4) befindlichen Menschen (2) vorhanden sind.

3. Entspannungsraum nach Anspruch 1 oder 2, **dadurch gekennzeichnet, dass** fest mit der Innenrandfläche (5) verbundene Liegemöbel (7) vorhanden sind.

4. Entspannungsraum nach einem der Ansprüche 1 bis 3, **dadurch gekennzeichnet, dass** die Innenrandfläche (5) stückweise aus transparentem und/oder transluzentem Material zusammengesetzt ist.

5. Entspannungsraum nach einem der Ansprüche 1 bis 4, **dadurch gekennzeichnet, dass** die Innenrandfläche (5) mit Wasser (8) aussenseitig umfänglich und/oder durch das Torusloch (9) umspült ist.

6. Entspannungsraum nach einem der Ansprüche 1 bis 5, **dadurch gekennzeichnet, dass** er frei schwimmend im Wasser (8) gelagert ist.

7. Entspannungsraum nach Anspruch 6, **dadurch gekennzeichnet, dass** ein sich ausbildender Wirbel (11) mit seinen Strömungsschlieren (12) an einen Teil der Innenrandfläche (5) projiziert ist.

8. Entspannungsraum nach Anspruch 7, **dadurch gekennzeichnet, dass** ein zur Aufnahme des Wirbels (11) ausgebildetes Unterwassermikrofon (11) vorhanden und geeignet steuernd mit den Einrichtungen (6) verbunden ist.

9. Verwendung eines Entspannungsraums nach einem der Ansprüche 1 bis 8 im professionellen Wohlfühl-Bereich.
